Europäisches Patentamt

(19) European Patent Office

Office européen des brevets

(11) Numéro de publication: **0 066 916**
**B1**

(12) FASCICULE DE BREVET EUROPEEN

(45) Date de publication du fascicule du brevet:
19.03.86

(51) Int. Cl.⁴: **A 61 C 19/04,** A 61 B 5/10

(21) Numéro de dépôt: 82200604.5

(22) Date de dépôt: 18.05.82

(54) Dispositif de mesure dynamique de la mobilité dentaire.

(30) Priorité: 26.05.81 FR 8110679

(43) Date de publication de la demande:
15.12.82 Bulletin 82/50

(45) Mention de la délivrance du brevet:
19.03.86 Bulletin 86/12

(84) Etats contractants désignés:
DE GB IT

(56) Documents cités:
US - A - 4 034 476

(73) Titulaire: Etablissement Public dit: CENTRE NATIONAL
DE LA RECHERCHE SCIENTIFIQUE (CNRS), 15, Quai
Anatole France, F-75700 Paris (FR)

(72) Inventeur: Bourdeau, Charles, REBIGUE,
F-31320 Castanet Tolosan (FR)
Inventeur: Fadel, René, REBIGUE, F-31320 Castanet
Tolosan (FR)
Inventeur: Benque, Edmond, 63, boulevard Carnot,
F-31000 Toulouse (FR)
Inventeur: Jonlot, Bernard, 27, rue de la Dalbade,
F-31000 Toulouse (FR)
Inventeur: Paloudier, Gérard, 8, allées François Verdier,
F-31000 Toulouse (FR)

(74) Mandataire: Barre, Philippe, Cabinet Barre, Gatti,
Laforgue 95, rue des Amidonniers, F-31069 Toulouse
Cedex (FR)

## Description

La présente invention a trait à un dispositif de mesure de la mobilité des dents.

Le retrait des gencives entraîne un accroissement de la mobilite des dents. Au delà d'un certain seuil, les dents doivent être traitées si l'on veut éviter leur chute à plus ou moins brève échéance. Afin de déterminer la thérapeutique la plus appropriée, puis d'en évaluer l'efficacité, il est nécessaire de mesurer la mobilité des dents concernées.

Or, actuellement, il n'existe pas d'appareil permettant une évaluation simple et précise de ce paramètre. Plusieurs dispositifs encombrants et coûteux ont été développés dans des centres de recherche mais ils sont difficilement utilisables, mêmes par des spécialistes (on pourra se reporter par exemple au brevet US n° 4034476). Le principe généralement adopté consiste en une mesure statique du déplacement de la dent lorsqu'elle est soumise à un effort constant appliqué perpendiculairement à sa face verticale externe. De tels appareils comprennent un dynamomètre et un comparateur. Etant donné que les déplacements mesurés sont très faibles (de quelques microns à quelques millimètres), ces appareils doivent être fixés de façon extrêmement rigide sur la mâchoire.

La complexité de ces appareils et leurdifficulté d'utilisation ont fait obstacle à leur développement industriel et l'appréciation de la mobilité des dents, à l'heure actuelle, est effectuée en pratique par estimation de leur déplacement après une excitation manuelle.

Cette imprécision de mesure rend illusoire toute référence à une échelle de mobilité permettant de définir, de façon sûre et précise, le traitement à appliquer dans chaque cas.

La présente invention a pour objectif de remédier aux insuffisances des techniques connues en indiquant un dispositif de mesure ne nécessitant pas de fixation sur la mâchoire.

Un autre objectif de la présente invention est de fournir un dispositif de mesure précis et fidèle, et ceci malgré des conditions de mesure parfois difficiles.

Un autre objectif de la présente invention est de fournir un dispositif de mesure d'un coût modique et de faible encombrement, ne nécessitant qu'une dextérité moyenne, normale et habituelle chez un praticien dentiste.

Un autre objectif de la présente invention est de permettre de se référer à une échelle de mobilité dentaire comportant au moins quatre plages que l'on peut définir, de façon non limitative, de la manière suivante:

- mobilité I: dents saines (déplacement inférieur à 0,1 mm).
- mobilité II: dents dont on peut apprécier le déplacement lorsqu'on les excite manuellement (déplacement compris entre 0,1 et 0,5 mm).
- mobilité III: dents très mobiles (déplacement compris entre 0,5 et 1 mm).

- mobilité IV: dents à extraire.

A cet effet, le dispositif de mesure visé par l'invention est doté d'un capteur adapté pour engendrer un signal électrique représentatif du déplacement provoqué d'une dent et de moyens d'affichage des résultats de mesure; selon la présente invention, ledit dispositif comprend:

. des moyens adaptés pour générer une energie cinetique determinée et pour la transmettre à une dent, en vue de provoquer le déplacement de ladite dent, ces moyens réalisantiltiledit déplacement de la dent,

. des moyens de verrouillage et de libération adaptés pour déclencher les moyens de génération en vue d'autoriser la production de l'énergie cinétique et sa transmission à la dent par l'entremise des moyens de transmission,

. des moyens de mesure de la variation d'amplitude du signal issu du capteur dans un laps de temps prédéterminé, adaptés pour délivrer aux moyens d'affichage un signal représentatif de cette variation d'amplitude au cours dudit laps de temps.

Les moyens de mesure sont en particulier adaptés pour générer un signal représentatif du déplacement de la dent pour amplifier ce signal et pour mesurer la variation d'amplitude du signal amplifié au cours d'un laps de temps constant, en particuller compris entre 0,1 et 1,5 ms et de préférence de l'ordre de 1 ms. On mesure en fait une tension proportionnelle au déplacement de la dent pendant ce laps de temps, donc proportionnelle à la vitesse moyenne du déplacement. Le laps de temps est choisi suffisamment long pour avoir une mesure significative, mais aussi suffisamment court pour éviter la prise en compte d'informationsparasites dues aux mouvements du bras de l'opérateur.

D'autres caractéristiques du dispositif de mesure conforme à la présente invention seront mises en évidence par la description ci-après, en regard des dessins annexés, donnés à titre d'exemple non limitatif et sur lesquels:

- la figure 1 represente une vue en coupe de la partie mecanique et électromécanique du dispositif de mesure selon la présente invention, en position armée,

- la figure 2 représente une vue en coupe de la même partie mais en position déclenchée,

- la figure 3 représente un schéma synoptique des circuits électroniques équipant le dispositif contorme a la presente invention,

- la figure 4 illustre un exemple de courbe du déplacement d'une dent en fonction du temps et,

-la figure 5 illustre la façon d'utilisier le dispositif selon l'invention pour effectuer la mesure dynamique de la mobilité d'une dent.

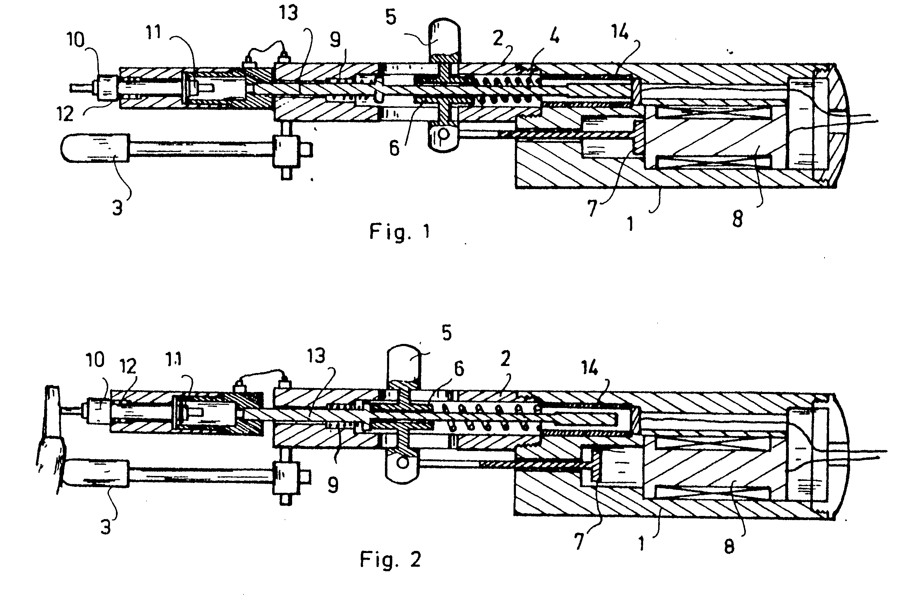

Le dispositif représenté à titre d'exemple aux figures 1 et 2 comprend un corps 2 constitué par un cylindre creux, à l'arrière duquel est fixé un manchon 1 qui sert au maintien du dispositif. A l'avant et à l'extétieur du corps 2 est fixé un embout 3 destiné à prendre appui sur la partie dure de la gencive en un point situé à l'aplomb de la dent à inspecter. Toutefois, cet embout ne

3 **0 066 916** 4

constitue qu'une aide au positionnement de l'appareil mais n'est nullement indispensable.

A l'interieur du cylindre creux 2 sont agences des moyens de génération d'une énergie cinétique comprenant un ressort taré 4 ainsi que des moyens d'armement de ce ressort 4 et des moyens de verrouillage desdits moyens d'armement. Ces moyens d'armement sont constitués par une gachette 5 solidaire d'un coulisseau 6 dont le déplacement engendre la compression du ressort taré 4. Egalement solidaire du coulisseau 6, un verrou 7 formant l'armature d'une electroaiment 8, est apte à maintenir le ressort 4 comprimé lorsque l'electroaimant 8 est excité. Le ressort 4 est taré au moyen d'un contre-ressort 9 dont la force de compression est réglable.

A l'avant du cylindre 2 sont agencés des moyens de libération de l'énergie cinétique dont un palpeur 10 que l'opérateur vient mettre en contact avec la dent à inspecter. Ce palpeur 10 est usiné intérieurement de manière à réaliser un micro-contact 11 de forme annulaire. Un petit ressort 12, très souple, maintient le palpeur 10 vers l'avant en position de repos, assurant la fermeture du micro-contact. Le micro-contact 11 sert de rupteur sur la ligne d'alimentation électrique de électroaimant 8 qui maintient l'armement.

Entre le palpeur 10 et le coulisseau 6 est agencé un piston 13 assurant la transmission de l'énergie cinétique qui lui est communiquée par le coulisseau 6 lorsque le ressort 4 est libéré.

Dés que le palpeur 10 entre en contact avec la dent à inspecter, le micro-contact s'ouvre et provoque l'ouverture du circuit d'alimentation de l'electroaimant 8, relachant le verrou 7. Le ressort 4 se détend entraînant le coulisseau 6 qui communique au piston 13 ladite énergie cinetique. Celle-ci transmise à la dent à inspecter par le piston 13, provoque le deplacement de la dent.

Sous l'action de l'impulstion transmise par le piston 13, la dent recule plus ou moins selon l'état des ligaments de la paroi alvéolaire. La vitesse avec laquelle recule la dent est significative de cet etat et la mesure de cette vitesse permet d'apprecier de façon precise la mobilité de la dent. Pour mesurer cette vitesse, on mesure son deplacement, pendant un laps de temps déterminé au moyen d'un capteur 14 magnétique, associe au piston 13. Ce capteur 14 délivre un signal electrique dont l'amplitude est proportionnelle au deplacement de la dent et par conséquent à sa vitesse moyenne si l'on mesure toujours cette amplitude à la fin d'un laps de temps constant.

Le contre-ressort 9, agencé entre la paroi frontale du corps 2 et une butée prévue sur le piston 13, non seulement assure le tarage du ressort 4 mais encore rappelle le piston 13 en position de repos (figure 1) après liberation de l'energie cinétique.

L'ensemble des elements mécanques et électromecaniques ci-dessus énumérés sont agencés dans un boîtier conforme de façon à présenter la forme générale et la taille approximative d'un stylographe.

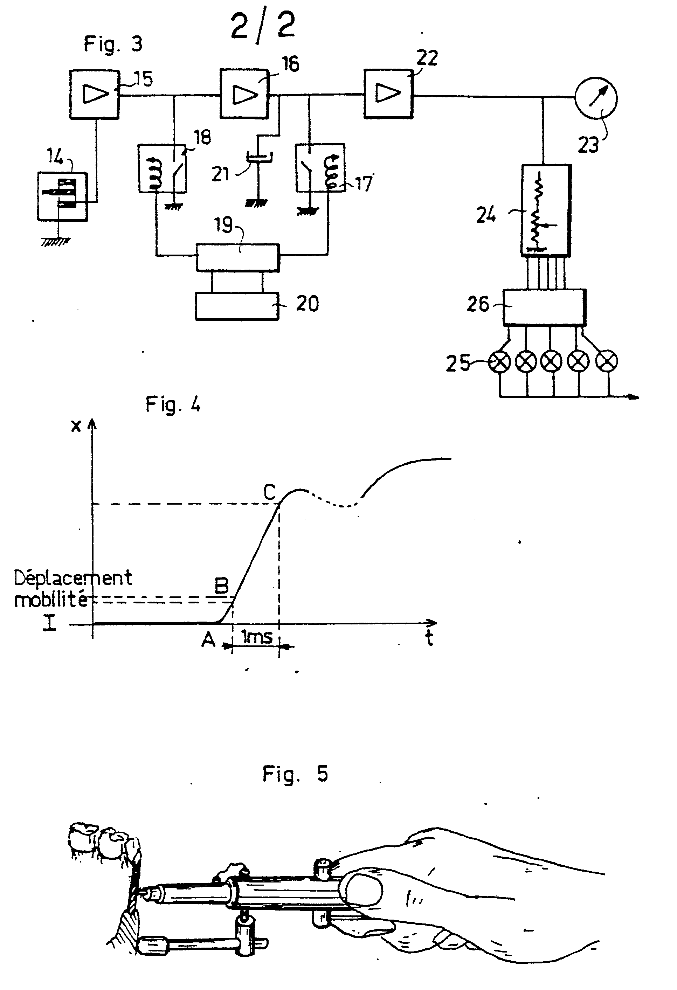

Sur la figure 3, on peut voir le dispositif electronique de traitement des informations données par le capteur magnétique 14. Ce dispositif comprend un amplificateur 15 adapte pour rendre exploitable le signal delivré par le capteur magnétique 14. Le signal amplifié est alors délivré à un amplificateur à maximum 16 qui, commandé par deux interrupteurs 17 et 18, permet de s'affranchir des mouvements de l'operateur et de minimiser l'erreur introduite par le recul de son bras.

L'interrupteur 17 est actionne par un detecteur à seuil 19 et remet à zéro un dispositif de memorisation de l'amplificateur à maximum 16 dès que le capteur 14 indique un deplacement legèrement inférieur à celui correspondant à une mobilité physiologique (mobilité I). Celui-ci déclenche egalement un monostable réglable 20. Le monostable 20 ouvre l'interrupteur 18 reliant l'entrée de l'amplificateur à maximum 16 à la masse. Le monostable 20 est réglé pour un temps correspondant au temps de réponse du système "dentpiston-ressort". Au bout de ce temps, le monostable bascule, fermant l'interrupteur 18 et commutant une tension nulle sur l'amplificateur à maximum 16 qui délivre alors au dispositif de mémorisation une tension proportionnelle au déplacement de la dent pendant le temps déterminé par le monostable 20, donc proportionnelle à la vitesse moyenne de déplacement pendant ce temps. Le dispositif de mémorisation est constitué par un condensateur 21 dont la charge accumulée pendant le temps déterminé par le monostable 20 est donc une fonction de la vitesse moyenne de déplacement de la dent pendant ce temps.

Cette tension est alors amplifiée dans un circuit 22 en vue soit de sa lecture directe sur un galvanomètre 23, soit de sa comparsision à des tensions de référence dans un circuit 24 pilotant un détecteur de seuils 26. Celuici commande l'alimentation de lampes témoins 25 correspondant chacune à une plage de mobilité.

Un exemple de l'allure de la courbe du déplacement d'une dent en fonction du temps, mesuré par le capteur magnétique 14, est donné à la figure 4. Sur cette figure, l'origine des temps correspond à l'ouverture du micro-contact 11. Dès que le piston 13 communique à la dent l'énergie cinétique engendrée par le ressort4 et le coulisseau 6 (point A), la dent commence à ce déplacer et avec elle le piston 13 et la partie mobile du capteur magnétique 14. Celui-ci délivre alors un signal au circuit électronique. Dès que l'amplitude de ce signal atteint un seuil (point B) légèrement inférieur à l'amplitude du signal correspondant à la mobilité I de l'échelle définie ci-avant, le détecteur de seuil 19 actionne l'interrupteur 17 et déclenche le monostable 20. C'est l'ori gine des temps de la mesure de la vitesse. Le condensateur 21 se charge alors jusqu'au moment où le monostable 20 bascule (point C) et ferme l'interrupteur 18. La charge du condensateur est donc bien fonction de la vitesse de déplacement de la dent dans le laps de temps déterminé par le monostable 20.

Cependant, la difficulté de la mesure réside dans le fait que l'energie cinetique transmise à la dent n'est pas absorbée en totalité par le recul de celle-ci. L'énergie cinétique non absorbée provoque le recul du bras de l'opérateur et la mesure au-delà du point C n'est plus du tout significative. Cette difficulté est levée, d'une part, grâce à un calibrage de la durée de la mesure de vitesse, correspondant au temps maximum permettant à une dent de mobilité la plus élevée (mobilité IV) d'atteindre son déplacenent maximum, et, d'autre part, grâce à un calibrage des moyens de oénération de l'énergie cinétique telle que la vitesse de recul du bras soit négligeable par rapport à celle de la dent. Ces expériences menées sur divers patients ont montré que la mesure à la volée, sans appui sur la gencive au moyen de l'embout 3, donne des résultats précis et fidèles. En particulier, le dispositif selon l'invention permet l'inspection des dents situées à l'arrière de la mâchoire et dont l'accès serait impossible avec un appareil plus encombrant. Il convient également de signaler que les mesures sont possibles en face avant comméen face arrière.

L'affichage de la mobilité peut se faire sur un boîtier séparé contenant les circuits électroniques ou directement sur le corps du capteur de mesure au moyen de diodes électroluminescentes ou d'afficheurs lumineux.

Pour que les mesures s'effectuent dans de bonnes conditions, il faut que le point d'impact du palpeur 10 soit situé à une distance constante du point de pivotement de la dent, de manière à exercer un couple constant sur celle-ci. Ce point est situe a peu près au 2/3 de la hauteur de la dent à partir de la gencive. Par ailleurs, un tampon antidérapant, fixé à l'extrémité du palpeur peut être prévu pour éviter un glissement latéral du dispositif.

Il est évident que la description qui précède n'a été donnée qu'à titre d'exemple non limitatif et de nombreuses modifications pourraient âtre apportées sans pour autant sortir du cadre de la présente invention. C'est ainsi que les moyens de libération de l'énergie cinétique pourraient être mécaniques, bien qu'une telle solution exige une plus grande dextérité de la part de l'opérateur. De même, le microcontact 11 pourrait être remplacé par une fenêtre opto-électronique moins fragile. De même on pourrait envisager tout moyen mécanique, électro-mécanique ou autre pour générer l'énergie cinétique nécessaire pour provoquer le déplacement de la dent.

## Revendications

1/ - Dispositif de mesure dynamique de la mobilité dentaire, ledit dispositif étant doté d'un capteur (14) adapté pour engendrer un signal électrique representatif du déplacement provoqué d'une dent et de moyens (23, 25) d'affichage des résultats de mesure et étant caractérisé en ce qu'il comprend:

. des moyens (4, 6, 13) adaptés pour générer une énergie cinétique déterminée et pour la transmettre a une dent, en vue de provoquer le déplacement de ladite dent, ces moyens réalisant ainsi ledit déplacement de la dent,

. des moyens (7, 11) de verrouillage et de libération adaptés pour déclencher les moyens (4, 6) de génération en vue d'autoriser la production de l'énergie cinétique et sa transmission à la dent par l'entremise des moyens de transmission (13),

. des moyens de mesure de la variation d'amplitude du signal issu du capteur (14) dans un laps de temps prédéterminé, adaptés pour délivrer aux moyens d'affichage un signal représentatif de cette variation d'amplitude au cours dudit laps de temps.

2/ - Dispositif de mesure dynamique selon la revendication 1, caractérisé en ce que les moyens de génération, d'énergie cinétique comprennent:

. un ressort (4) taré, agencé pour être apte à entraîner un coulisseau (6) lors de sa détente et,

. des moyens (5) d'armement du ressort (4) adaptés pour le mettre sous tension en vue de générer ladite énergie cinétique.

3/ - Dispositif de mesure dynamique selon la revendication 2, caractérisé en ce que le ressort (4) est taré par réglage de la tension d'un contre-ressort (9).

4/ - Dispositif de mesure dynamique selon la revendication 2, caractérisé en ce que les moyens d'armement comprennent une gâchette (5) reliée au coulisseau (6) dont le déplacement met le ressort (4) sous tension.

5/ - Dispositif de mesure dynamique selon la revendication 2, caractérisé en ce que les moyens de verrouillage comprennent un électroaimant (8) à armature (7) solidaire du coulisseau (6).

6/ - Dispositif de mesure dynamique selon la revendication 5, caractérisé en ce que les moyens de libération comprennent des moyens (11) de coupure de l'alimentation de l'électroaimant (8).

7/ - Dispositif de mesure dynamique selon la revendication 6, caractérisé en ce que les moyens de coupure de l'alimentation de l'électroaimant (8) comprennent un micro-contact (11) actionné par les moyens de transmission.

8/ - Dispositif de mesure dynamique selon la revendication 7, caractérisé en ce que les moyens de transmission comprennent un piston (13) adapté pour recevoir l'énergie cinétique engendrée par le ressort (4) entraînant le coulisseau (6), et un palpeur (10) agencé pour venir en contact avec une dent, d'une part pour actionner le microcontact (11) entraînant la coupure de l'alimentation de l'électroaimant (8) lors de son appui contre la dent, et d'autre part, pour recevoir l'énergie cinétique communiquée par le piston (13) et pour la transmettre à son tour à la dent.

9/ - Dispositif de mesure dynamique selon la revendication 8, dans lequel les moyens de mesure comprennent un capteur magnétique (14), solidaire du piston (13) et adapté pour délivrer à ces circuits électroniques un signal électrique représentatif du déplacement dudit piston.

10/ - Dispositif de mesure dynamique selon la revendication 9, caractérisé en ce que les circuits électroniques comprennent un amplificateur à maximum (16) et deux interrupteurs (17, 18) commandés par un détecteur de seuil (19) et un monostable (20) en vue de délivrer aux moyens d'affichage (23, 25) un signal représentatif du déplacement de la dent pendant un laps de temps déterminé par le monostable (20).

11/ - Dispositif de mesure dynamique selon l'une des revendications 1 à 10 dans lequel les moyens d'affichage comprennent un galvanomètre (23).

12/ - Dispositif de mesure dynamique selon les revendications 1 à 11, caractérisé en ce que les moyens d'affichage comprennent une pluralité de circuits (24) de comparaison du signal représentatif du déplacement de la dent pendant ledit laps de temps, à des tensions de référence définissant chacune une plage de mobilité, en vue d'alimenter des témoins (25) correspondant chacun à l'une desdites plages de mobilité.

13/ - Dispositif de mesure dynamique selon l'une des revendications 1 à 12, caractérisé en ce que les moyens de génération et de transmission de l'énergie cinetique, les moyens de verrouillage et de libération de ladite énergie cinétique et éventuellement les moyens d'affichage sont agencés dans un boîtier conformé de façon à présenter la forme générale et la taille approximative d'un stylographe.

## Claims

1/ Apparatus for measuring tooth mobility, the said apparatus being provided with a sensor (14) designed to generate an electrical signal representing the caused displacement of a tooth and with devices (23, 25) for displaying the measurement findings and characterized by comprising:
- devices (4, 6, 13) designed to generate a determined kinetic energy and transmit it to a tooth with a view to causing the displacement of the said tooth, these devices also carrying out the said displacement of the tooth,
- locking and releasing devices (7,11) designed to trigger generating devices (4, 6) with a view to authorizing the production of kinetic energy and its transmission to the tooth by means of the transmission devices (13),
- devices to measure the variation in amplitude of the signal emitted by the sensor (14) in a predetermined lapse of time, designed to provide the display devices with a signal representative of this amplitude variation during the said lapse of time.

2/ Apparatus as claimed in Claim 1, characterized by kinetic energy generation devices comprising.:
- a calibrated spring (4) mounted so as to drive a slider (6) upon its extension and,
- spring (4) arming devices (5) designed to compress it in order to generate the said kinetic energy.

3/ Apparatus as claimed in claim 2, characterized by the spring (4) being calibrated by adjustment of the tension of a counter-spring (9)

4/ Apparatus as claimed in claim 2, characterized by the arming devices comprising a trigger (5) connected to the slide (6) whose displacement compresses the spring (4).

5/ Apparatus as claimed in claim 2, characterized by the locking devices comprising an electromagnet (8) with a core (7) joined to the slider (6).

6/ Apparatus as claimed in claim 5, characterized by the release devices comprising the power supply disconnect devices (11) of the electromagnetic (8).

7/ Apparatus as claimed in claim 6, characterized by the power supply disconnect devices of the electromagnet (8) comprising a microswitch (11) activated by the transmission devices.

8/ Apparatus as claimed in claim 7, characterized by the transmission devices comprising a piston (13) designed to receive the kinetic energy generated by the spring (4) driving the slider (6), and a feeler (10) mounted so as to engage a tooth in order, on one hand, to activate the microswitch (11) causing the disconnection of the electromagnet's (8) power supply as it rests against the tooth and, on the other hand, to receive the kinetic energy transmitted by the piston (13) and convey it in turn to the tooth.

9/ Apparatus as claimed in claim 8, wherein the measurement devices comprise a magnetic sensor (14) joined to the piston (13) and designed to deliver an electrical signal representative of the travel of the said piston to these electronic circuits.

10/ Apparatus as claimed in claim 9, characterized by the electronic circuits comprising a maximum amplifier (16) and two switches (17, 18) controlled by a threshold detector (19) and a monostable (20) so as to deliver to the display devices (23, 25) a signal representative of the displacement of the tooth during a lapse of time determined by the monostable (20).

11/ Apparatus as claimed in any one of claims 1 through 10 wherein the display devices comprise a galvanometer (23).

12/ Apparatus as claimed in claims 1 through 11, characterized by the display devices comprising several comparison circuits (24) for the signal representative of the displacement of the tooth, during the said lapse of time, at reference voltages which each define a range of motion with a view to supplying indicators (25) each corresponding to one of the said ranges of motion.

13/ Apparatus as claimed in any one of claims 1 through 12, characterized by the kinetic energy generation and transmission devices, the locking and releasing devices of the said kinetic energy, and possibly the display devices, are mounted in a compliant box so as to have the overall shape and approximate size of a stylograph.

## Patentansprüche

1. Gerät um die Beweglichkeit von Zähnen zu messen, versehen mit einem Fühler (14), der ein elektrisches Signal erzeugt, das der hervorgerufenen Lageveränderung eines Zahnes entspricht, sowie mit Vorrichtungen (23, 25) zur Anzeige der Messergebnisse, mit folgenden Merkmalen:
   - geeignete Vorrichtungen (4, 6, 13) zur Erzeugung einer festgelegten kinetischen Energie und zur Übertragung derselben auf einen Zahn, mit dem Ziel, die Lage dieses Zahnes zu verändern, aus diese Weise bewirken diese Vorrichtungen die besagte Lagerveränderung des Zahnes;
   - Ver- und Entriegelungsvorrichtungen (7, 11) zur Auslösung der Energieerzeugungsvorrichtungen (4, 6), um die kinetische Energie zu erzeugen und sie mit Hilfe von Ubertragungsvorrichtungen (13) auf den Zahn zu übertragen;
   - Vorrichtungen zum Messen der Amplitudenänderung des von dem Fühler (14) erzeugten Signals innerhalb eines festgelegten Zeitabschnitts, die mit Hilfe von Anzeigevorrichtungen ein dieser Amplitudenänderung innerhalb des besagten Zeitabschnitts entsprechendes Signal liefern.

2. Gerät gemäss Anspruch 1, dadurch gekennzeichnet, dass sich die Vorrichtungen zur Erzeugung der kinetischen Energie aus folgenden Teilen zusammensetzen:
   - einer geeichten Feder (4), die so angebracht ist, dass sie bei ihrer Rückfederung einen Schieber (6) in Bewegung setzt, und
   - Vorrichtungen (5) zum Spannen der Feder (4), um die besagte kinetische Energie zu erzeugen.

3. Gerät gemäss Anspruch 2, dadurch gekennzeichnet, dass die Feder (4) durch die Einstellung der Spannung einer Gegenfeder (9) austariert wird.

4. Gerät gemäss Anspruch 2, dadurch gekennzeichnet, daß die Vorrichtung zum Spannen der Feder (4) einen Drücker (5) beinhaltet, der mit dem Schieber (6) verbunden ist und dessen Lageveränderung die Feder (4) spannt.

5. Gerät gemäss- Anspruch 2, dadurch gekennzeichnet, daß die Verriegelungsvorrichtung einen Elektromagneten (8) mit Magnetanker (7) beinhaltet, der mit dem Schieber (6) fest verbunden ist.

6. Gerät gemäss Anspruch 5, dadurch gekennzeichnet, daß die Entriegelungsvorrichtung eine Vorrichtung (11) zur Unterbrechung der Stromversorgung des Elektromagneten (8) beinhaltet.

7. Gerät gemäss Anspruch 6, dadurch gekennzeichnet, daß die Vorrichtung zur Unterbrechung der Stromversorgung des Elektromagneten (8) einen von der Übertragungsvorrichtung betätigten Kleinkontakt (11) beinhaltet.

8. Gerät gemäss Anspruch 7, dadurch gekennzeichnet, daß die Übertragungsvorrichtung einen Kolben (13) besitzt, der die von der den Schieber (6) betätigenden Feder (4) ausgelöste kinetische Energie übernimmt, sowie einen Fühler (10), der so angebracht ist, daß er Kontakt mit einem Zahn hat, einerseits um den Kleinkontakt (11) zu betätigen und so die Stromversorgung des Elektromagneten (8) zu unterbrechen, wenn er an den Zahn stößt, und andererseits um die von dem Kolben (13) übermittelte kinetische Energie zu übernehmen und sie seinerseits an den Zahn weiterzuleiten.

9. Gerät gemäss Anspruch 8, deren Messvorrichtung einen magnetischen Fühler (14) besitzt, der mit dem Kolben (13) fest verbunden ist und den elektronischen Schaltkreisen ein der Lageveränderung dieses Kolbens entsprechendes Signal liefert.

10. Gerät gemäss Anspruch 9, dadurch gekennzeichnet, daß die elektronischen Schaltkreise einen Maximalverstärker (16) und zwei durch einen Schwellenwertdetektor (19) und eine monostabile Schaltung (20) betätigte Schalter besitzen, um den Anzeigevorrichtungen (23, 25) ein der Lageänderung des Zahnes innerhalb eines durch die monostabile Schaltung (20) festgelegten Zeitraums entsprechendes Signal zu liefern.

11. Gerät gemäss einem der Ansprüche 1 bis 10, deren Anzeigevorrichtungen einen Galvanometer (23) beinhalten.

12. Gerät gemäss den Ansprüchen 1 bis 11, dadurch gekennzeichnet, daß die Anzeigevorrichtungen eine ganze Reihe Schaltkreise (24) besitzen, die das der Lageänderung des Zahnes innerhalb eines bestimmten Zeitraumes entsprechende Signal mit Bezugsspannungen vergleichen, von denen jede einem bestimmten Beweglichkeitsbereich entspricht; hierdurch werden Kontrolleuchten (25) in Gang gesetzt, von denen jede einem dieser Beweglichkeitsbereiche entspricht.

13. Gerät gemäss einem der Ansprüche 1 bis 12, dadurch gekennzeichnet, daß die Vorrichtungen zur Erzeugung und Übertragung der kinetischen Energie, zur Verriegelung und Freigabe dieser kinetischen Energie sowie gegebenenfalls die Anzeigevorrichtungen in einem Gehäuse untergebracht sind, dessen allgemeine Form und Größe ungefähr der eines Stylographen entsprechen.

Fig. 1

Fig. 2

Fig. 3

Fig. 4

Déplacement
mobilité

Fig. 5